# EUROPÄISCHE PATENTSCHRIFT

(11) **EP 2 553 495 B1**
(45) Veröffentlichungstag und Bekanntmachung des Hinweises auf die Patenterteilung: **23.08.2017**
(21) Anmeldenummer: 11804493.2
(22) Anmeldetag: 12.12.2011
(51) Int. Cl.: G01T 1/29

(54) **RÖNTGENZEILENDETEKTOR SOWIE VERFAHREN ZUR HERSTELLUNG DESSELBEN**
X-RAY LINE DETECTOR AND METHOD FOR THE PRODUCTION THEREOF
DÉTECTEUR DE LIGNES À RAYONS X AINSI QUE PROCÉDÉ DE FABRICATION DUDIT DÉTECTEUR DE LIGNES À RAYONS X

(30) Priorität: 13.12.2010 DE 102010054340
(43) Veröffentlichungstag der Anmeldung: 06.02.2013
(73) Patentinhaber: YXLON International GmbH, 22419 Hamburg (DE)
(72) Erfinder: BAVENDIEK, Klaus, 22851 Norderstedt (DE)
(74) Vertreter: DTS Patent- und Rechtsanwälte Schnekenbühl und Partner mbB
(86) Internationale Anmeldenummer: PCT/EP2011/006260
(87) Internationale Veröffentlichungsnummer: WO 2012/079737

(56) Entgegenhaltungen:
- DE-A1-102005 014 187
- GB-A- 2 005 405
- US-A- 4 415 808
- US-A1- 2006 054 832

## Beschreibung

Die Erfindung befasst sich mit einem Röntgenzeilendetektor mit einer vorgegebenen Anzahl von Trägermodulen, die jeweils eine Photodiode aufweisen und in einem Gehäuse angeordnet sind. Darüber hinaus befasst sich die Erfindung mit einem Verfahren zur Herstellung eines solchen Röntgenzeilendetektors.

Aus der DE 10 2005 014 187 A1 ist ein Röntgendetektor mit einer vorgegebenen Anzahl von Trägermodulen bekannt, die jeweils die gleiche Breite aufweisen. Auf jedem Trägermodul ist eine einstückige Leiterplatine angebracht, die breiter ist als die aktive Fläche der eine Photodiode bildeneden Pixel. Auf jeder Photodiode ist jeweils ein Szintilalatorelement aufgebracht. Der Röntgendetektor ist als ein Array von Trägermodulen aufgebaut, so dass sich ein flächenförmiger Röntgendetektor ergibt. Die Form jeder Leiterplatine ist an die Form des zugehörigen Trägermoduls angepasst. Auf jeder Leiterplatine ist ein zweidimensionales Feld von Photodioden, ein Photodiodenarray, angeordnet. Auf dem Photodiodenarray ist ein zugeordnetes Szintillatorarray aufgebracht. Darauf ist ein Kollimator angebracht. Anstatt der Kombination aus Photodiodenarray und Szintillatorarray kann auch ein Array aus Detektorelementen verwendet werden, die die auftreffende Röntgenstrahlung direkt in elektrische Signale umsetzen, ohne einen Szintillator zu benötigen.

Aus der GB 2 005 405 A ist ein Strahlungsdetektor bekannt, der zwei gegenüberliegende Trägermodule mit jeweils darauf angebrachten Zeilen von Photodioden aufweist, wobei auf jeder Photodiode ein Szintillator angebracht ist. Der Abstand zwischen nebeneinanderliegenden Photodioden samt Szintillatoren ist so, dass in diese Lücken jeweils ein Szintillator des jeweils anderen, gegenüberliegenden Trägermoduls eingeschoben ist. Darüber hinaus ist zwischen jeweils zwei nebeneinanderliegenden Szintillatoren ein Zwischenraum ausgebildet, der durch ein für sichtbare Strahlung und Röntgenstrahlung undurchlässiges Material ausgefüllt ist.

Bei Röntgenzeilendetektoren, die insbesondere für CT-Verfahren eingesetzt werden, können für eine äquidistante Anordnung der Pixel der Photodioden der gesamten Zeile die Module mit den Photodioden nicht bündig aneinander gereiht werden, da sie Randbereiche aufweisen, in denen keine aktiven Bereiche der Photodioden ausgebildet sind. Werden solche Module dennoch bündig aneinander gereiht, so ergibt sich aufgrund der mangelnden Äquidistanz der Pixel der Photodioden auf benachbarten Modulen eine Verzerrung im Röntgenbild. Dies wurde teilweise akzeptiert, obwohl dadurch bei der Rekonstruktion Artefakte im Bild entstehen, was die Bildauswertung erschwert.

Aufgabe der Erfindung ist es deshalb, einen Röntgenzeilendetektor sowie sein Herstellungsverfahren zur Verfügung zu stellen, bei dem die Photodioden so angeordnet sind, dass auch bei einander angrenzenden Photodioden der Übergang nicht die oben geschilderten Verzerrungen im Röntgenbild bewirkt, ohne dass eine aufwendige Feinjustierung nötig ist.

Die Aufgabe wird durch einen Röntgenzeilendetektor mit den Merkmalen des Patentanspruches 1 gelöst. Dabei weist ein Szintillatorelement eine Reihe von Szintillatorblöcken samt jeweils dazwischen befindlicher, lichtundurchlässiger Schicht auf. Bevorzugt ist die Breite eines Szintillatorblocks gleich der Breite einer aktiven Fläche eines Pixels der Photodiode und die Breite einer lichtundurchlässigen Schicht gleich des Abstands der Zwischenräume zwischen den aktiven Bereichen benachbarter Pixel der Photodiode. Dadurch, dass die Szintillatorelemente, die auf den Photodioden angebracht sind und jeweils genau die Breite der aktiven Fläche der die Photodioden bildenden Pixel zuzüglich eines Zwischenraums zwischen zwei benachbarten Pixeln der Photodiode überdecken, können die Pixel des gesamten Röntgenzeilendetektors über dessen gesamte Länge äquidistant zueinander geordnet werden. Dies geschieht dadurch, dass die Trägermodule in zwei Reihen im Gehäuse angeordnet sind, wobei immer abwechselnd ein Trägermodul zum nächsten, jeweils auf der gegenüberliegenden Reihe angeordnet ist und die jeweiligen Szintillatorelemente dieser sich gegenüberliegender Trägermodule an ihren jeweiligen Stirnseiten aneinanderstoßen. Da die Szintillatorelemente genau die Breite der aktiven Fläche der Photodiode zuzüglich eines Abstands zwischen zwei benachbarten Pixeln haben, wird somit gewährleistet, dass zwei aneinander anschließende Photodioden ohne eine Änderung des Abstandes zwischen den Randpixeln dieser beiden Photodioden im Vergleich zu den Abständen der einzelnen Pixel innerhalb der Dioden aneinander anschließen. Dies ist dadurch möglich, dass die Trägermodule und somit auch die darauf angeordneten Leiterplatinen, die die Photodioden tragen, höchstens doppelt so breit sind wie ein Szintillatorelement. Dadurch wird sichergestellt, dass die Szintillatorelemente von sich jeweils gegenüberliegenden Trägermodulen auch tatsächlich aneinander anstoßen können, was nicht möglich wäre, wenn die Trägermodule mehr als doppelt so breit wie die Szintillatorelemente wären, da dann der Abstand zwischen zwei Szintillatorelementen von auf einer Reihe befindlichen Trägermodulen, die aneinander angrenzen, größer als die Länge des Szintillatorelements wäre. Dadurch würden die Randpixel von aneinander angrenzenden Photodioden nicht mehr denselben Abstand zueinander haben, wie die Pixel innerhalb einer Photodiode. Bevorzugt ist es wegen der weiter unten noch aufgeführten Gründe, wenn die Breite jedes Trägermoduls möglichst nahe an die doppelte Länge eines Szintillatorelements herankommt.

Eine vorteilhafte Weiterbildung der Erfindung sieht vor, dass das Gehäuse ein erstes Trägerelement und ein dazu spiegelsymmetrisch angeordnetes und ausgebildetes zweites Trägerelement aufweist, zwischen denen ein Eintrittsschlitz für Röntgenstrahlung ausgebildet ist, wobei die Trägermodule auf diesen beiden Trägerelementen alternierend angeordnet sind und die Szintillatorelemente in der Verlängerung des Eintrittsschlitzes angeordnet sind. Dadurch wird in besonders einfacher mechanischer Art und Weise eine Anordnung der Trägermodule innerhalb des Gehäuses gewährleistet und darüber hinaus auch noch ein Eintrittsschlitz für die Röntgenstrahlung geschaffen. Die räumliche Anordnung von Eintrittsschlitz und Szintillatorelementen ist so, dass die Photodioden zwangsläufig senkrecht hierzu links und rechts von den Szintillatorelementen angeordnet sind und somit nur senkrecht zur Einfallsrichtung der Röntgenstrahlung entstehende Szintillatorblitze registriert werden. Die durchgehende Röntgenstrahlung trifft dann nicht die Leiterplatinen. Damit auch am Szintillatorkristall gestreute Röntgenstrahlung die Leiterplatine nicht trifft, wird eine unten noch näher beschriebene Weiterbildung der Erfindung verwendet.

Eine weitere Weiterbildung der Erfindung sieht vor, dass die Trägerelemente L-förmig ausgebildet sind und in jedem Trägerelement jeweils eine parallel zum Eintrittsschlitz ausgerichtete Nut ausgebildet ist, in die formschlüssig eine an den Trägermodulen ausgebildete Nase eingreift. Dadurch ist es möglich, zwei Freiheitsgrade für die Anordnung der Trägermodule an den Trägerelementen festzulegen. Somit ist nur noch ein Verschieben der Trägermodule entlang der Schienen und parallel zum Eintrittsschlitz möglich. Dies erleichtert die Feinjustierung der Trägermodule und somit der Photodioden zueinander erheblich. Für den Fachmann ist es ohne weiteres klar, dass auch eine umgekehrte Ausbildung von der Erfindung umfasst ist - also wenn an jedem Trägerelement mindestens eine Schiene ausgebildet ist, die jeweils in mindestens eine dazu korrespondierende Ausnehmung an den Trägermodulen eingreift.

Eine weitere vorteilhafte Weiterbildung sieht vor, dass jedes Trägermodul über mindestens ein Festlegeelement, insbesondere eine Festlegeschraube, mit dem jeweiligen Trägerelement verbunden ist. Damit ist es möglich, nach der hochpräzisen Ausrichtung der einzelnen Trägermodule zueinander - die mittels der unten noch näher ausgeführten Methode zur Herstellung eines erfindungsgemäßen Röntgenzeilendetektors erfolgt - diese in ihrer Lage zum Gehäuse festzulegen. Dadurch ist es möglich, die zuvor während des Zusammenbaus des Röntgenzeilendetektors unter Druck stehenden Szintillatorelemente wieder von diesem Druck zu entlasten.

Eine weitere vorteilhafte Weiterbildung der Erfindung sieht vor, dass an den Enden des Gehäuses jeweils eine Abschlussplatte angeordnet ist, wobei die erste Abschlussplatte als ein Anschlag und die zweite Abschlussplatte als ein Spannelement ausgebildet sind. Dadurch wird ein weiterer Freiheitsgrad bezüglich der Anordnung und Ausrichtung der einzelnen Trägermodule beschränkt und die Möglichkeit gegeben, mittels der Spannelemente die Feinjustierung der einzelnen Trägermodule - und somit der Photodioden - zueinander vorzunehmen.

Eine weitere vorteilhafte Weiterbildung der Erfindung sieht vor, dass die zweite Abschlussplatte drei Spannschrauben aufweist, von denen die erste Spannschraube mit der ersten Reihe von Trägermodulen, die zweite Spannschraube mit der zweiten Reihe von Trägermodulen und die dritte Spannschraube mit den Szintillatorelementen zusammenwirken. Durch die Verwendung von Spannschrauben lässt sich eine sehr feine und sichere Einstellung der räumlichen Beziehungen zwischen den beiden Reihen von Trägermodulen und deren Szintillatorelementen zueinander - und somit also der Photodioden zueinander - einstellen.

Eine weitere vorteilhafte Weiterbildung der Erfindung sieht vor, dass das Gehäuse an seiner dem Eintrittsschlitz gegenüberliegenden Seite eine Deckelplatte aufweist. Dadurch wird die mechanische Stabilität des gesamten Röntgenzeilendetektors erhöht, da sein Gehäuse an allen Seiten - bis auf den Eintrittsschlitz - abgeschlossen ist. Darüber hinaus werden auch schädliche Umwelteinflüsse, beispielsweise durch Verschmutzung, auf die Leiterplatinen, die innerhalb des Gehäuses auf den Trägermodulen angeordnet sind, vermieden und eine Lichtdichtigkeit erreicht. Hierzu ist auch der Eingangschlitz mit einer Metallfolie lichtdicht und gegen elektromagnetische Strahlung versiegelt. Zusätzlich ist das ganze Gehäuse auch gegen Luftfeuchtigkeit abgeriegelt, damit sich die Szintillatorelemente nicht auf- oder ablösen.

Bevorzugt ist die Deckelplatte aus einem Material, das bei der verwendeten Energie der Röntgenstrahlung kaum Streustrahlung produziert, insbesondere ist es aus Carbon. Unter "kaum Streustrahlung produziert" ist zu verstehen, dass in der Deckelplatte bei der verwendeten Energie (in der Regel >> 100 keV) ein Anteil von unter 0,1 % der einfallenden Röntgenstrahlung absorbiert bzw. gestreut wird. Ein Kriterium wäre z.B. eine niedrige Ordnungszahl von Z < 10. Beispielsweise haben 5 mm Carbon bei 15 keV circa 50 % Absorption; werden Energien größer als 50 keV verwendet, geht quasi die gesamte Röntgenstrahlung mit einem zu vernachlässigenden Bruchteil an Absorption und somit Produktion von Streustrahlung hindurch.

Eine weitere vorteilhafte Weiterbildung der Erfindung sieht vor, dass zwischen der ersten Abschlussplatte und einer der beiden Reihen von Trägermodulen ein erstes Pufferstück eingesetzt ist, das halb so breit wie die Trägermodule ist, und zwischen der ersten Abschlussplatte und den Szintillatorelementen ein erstes Distanzstück eingesetzt ist das halb so breit wie die Szintillatorelemente ist. Dadurch wird gewährleistet, dass wenn das erste Trägermodul an der ersten Abschlussplatte anstößt, das ihm gegenüber liegende zweite Trägermodul mittels des Pufferstücks ebenfalls an der Abschlussplatte anschlägt, wenn die beiden Szintillatorelemente dieser beiden Trägermodule aneinander anschlagen. Um dem Druck des zweiten Szintillatorelements auf das erste Szintillatorelement entgegenzuwirken, ist zwischen dem ersten Szintillatorelement und der ersten Abschlussplatte das halb so breite Pufferstück angeordnet, das bevorzugt eine geringe Elastizität aufweist, wie zum Beispiel Hartgummi, damit es unter dem Druck des zweiten und der weiteren Szintillatorelemente auf das erste Szintillatorelement durch dieses geringfügig deformiert werden kann. Alternativ könnte das Pufferstück aus Metall sein. Dann ist es ein wenig schmaler ausgebildet und am Ende ist dann ein dünner Gummi- beziehungsweise Schaumstoff-Puffer zusätzlich angebracht.

Eine weitere vorteilhafte Weiterbildung der Erfindung sieht vor, dass zwischen der zweiten Abschlussplatte und einer der beiden Reihen von Trägermodulen ein zweites Pufferstück eingesetzt ist, das halb so breit wie die Trägermodule ist, und zwischen der zweiten Abschlussplatte und den Szintillatorelementen ein zweites Distanzstück eingesetzt ist, das halb so breit wie die Szintillatorelemente ist. Dadurch wird erreicht, dass die ansonsten stark unterschiedlich weit von der zweiten Abschlussplatte entfernten letzten Trägermodule jeder Reihe sowie das auf ungefähr halbem Weg dazwischen liegende letzte Szintillatorelement ungefähr denselben Abstand zu der zweiten Abschlussplatte aufweisen. Dadurch kann die Justierung der einzelnen Trägermodule zueinander einfacher vorgenommen werden. Ansonsten müssten stark unterschiedliche Spannelemente, insbesondere Spannschrauben, verwendet werden, um dies erreichen zu können.

Eine weitere vorteilhafte Weiterbildung der Erfindung sieht vor, dass die Leiterplatinen abgeknickt sind und in dem Bereich, der auf der anderen Seite des Knickbereichs in Bezug auf die Photodiode ausgebildet ist, elektronische Bauteile angeordnet sind. Dadurch wird - wie oben schon kurz angedeutet - erreicht, dass Röntgenstrahlung, die durch den Eintrittsschlitz in den Röntgenzeilendetektor eintritt und an dem Szintillatorelement gestreut wird, nicht auf die Leiterplatinen in einem Bereich auftreffen kann, in dem elektronische Bauteile auf diesem angeordnet sind. Dadurch wird eine Beschädigung beziehungsweise Beeinflussung der elektronischen Bauteile vermieden, die äußerst empfindlich auf Röntgenstrahlung reagieren.

Die Aufgabe wird auch durch ein Verfahren mit den Merkmalen des Patentanspruches 12 gelöst. Anhand eines solchen Herstellungsverfahrens wird ein Röntgenzeilendetektor erhalten, der zum einen äußerst exakte Abstände zwischen den Randpixeln benachbarter Photodioden aufweist, da eine äußerst exakte mechanische Ausrichtung der Trägermodule zueinander erfolgen kann. Zum anderen kann diese Ausrichtung in äußerst einfacher Art und Weise durch einfache mechanische Mittel erreicht werden, wobei es nicht auf eine hohe Genauigkeit der verwendeten Leiterplatinen ankommt. Deren Breite kann eine relativ hohe Streuung aufweisen, da sie bezüglich aneinander anschlagender Szintillatorelemente (beziehungsweise der darunter befindlichen Photodioden) keinen Einfluss auf den Abstand zwischen den Randpixeln aneinander angrenzender Photodioden haben. Wegen der höchstens doppelten Breite der Leiterplatinen samt des dazugehörigen Trägermoduls, sind sie nie breiter als die durch die Szintillatorelemente vorgegebenen Höchstabstände.

Eine vorteilhafte Weiterbildung des Verfahrens sieht vor, dass im Anschluss die einzelnen Trägermodule mittels Festlegemitteln, insbesondere mit Festlegeschrauben, am Gehäuse festgelegt werden und danach die dritte Spannschraube gelockert wird, so dass auf die Szintillatorelemente kein Druck mehr wirkt. Dadurch wird gewährleistet, dass die Spannung auf die Szintillatorelemente - die unter Spannung zueinander auf Anschlag zueinander gebracht werden müssen - nach der endgültigen Justierung sämtlicher Trägermodule zueinander wieder weggenommen werden kann, da die einzelnen Trägermodule nun in ihrer räumlichen Lage zu dem Gehäuse festgelegt sind und somit kein Druck mehr auf die Szintillatorelemente ausgeübt werden muss, wie dies davor für die genau Einstellung des Abstandes der Photodioden notwendig war. Dadurch wird vermieden, dass die Szintillatorelemente von den Photodioden, mit denen sie verklebt sind, abbrechen.

Eine weitere vorteilhafte Weiterbildung des erfindungsgemäßen Verfahrens sieht vor, dass anschließend die Deckelplatte mit dem Gehäuse verbunden, insbesondere verschraubt wird. Dadurch wird eine - wie oben schon beschriebene - weitere mechanische Versteifung und somit Stabilisierung des gesamten Gehäuses und somit des gesamten Röntgenzeilendetektors erreicht. Außerdem kann ein solcher Röntgenzeilendetektor auch "andersherum" in eine Röntgenprüfanlage eingebaut werden, so dass die Röntgenstrahlung nicht durch den Eintrittsschlitz, sondern aus der entgegengesetzten Richtung, also durch die Deckelplatte hindurchtritt. Dabei muss ein Material der Deckelplatte verwendet werden, das möglichst wenig Streustrahlung erzeugt, wie beispielsweise Carbon. Der nötige Eintrittsschlitz und die ebenfalls notwendige Abschirmung für die elektronischen Bauteile wird durch eine Vorrichtung vor der Abdeckplatte gebildet, die unabhängig vom Röntgenzeilendetektor, wenn auch äußerst genau auf dessen Geometrie abgestellt, auf Anwenderseite zur Verfügung gestellt wird.

Weitere Einzelheiten und Vorteile der Erfindung werden anhand der in den Figuren dargestellten und im Folgenden beschriebenen Ausführungsbeispiele erläutert. Es zeigen:
- Figur 1: einen Querschnitt durch ein erfindungsgemäßes Trägermodul beziehungsweise Ansichten eines erfindungsgemäßen Trägermoduls aus verschiedenen Blickrichtungen,
- Figur 2: eine Ansicht von oben auf einen montierten Röntgenzeilendetektor,
- Figur 3: einen Längsschnitt in einer Ebene, die parallel zu der in Figur 2 gezeigten Ebene ist,
- Figur 4: einen Querschnitt durch den Röntgenzeilendetektor der Figur 2, in derselben Richtung geschnitten wie Figur 1a,
- Figur 5: eine perspektivische Ansicht des fertig montierten Röntgenzeilendetektors von schräg oben ohne Deckelplatte und
- Figur 6: einen Querschnitt durch ein zweites Ausführungsbeispiel mit anderem Knickwinkel der Leiterplatine in einer Ebene wie Figur 4.

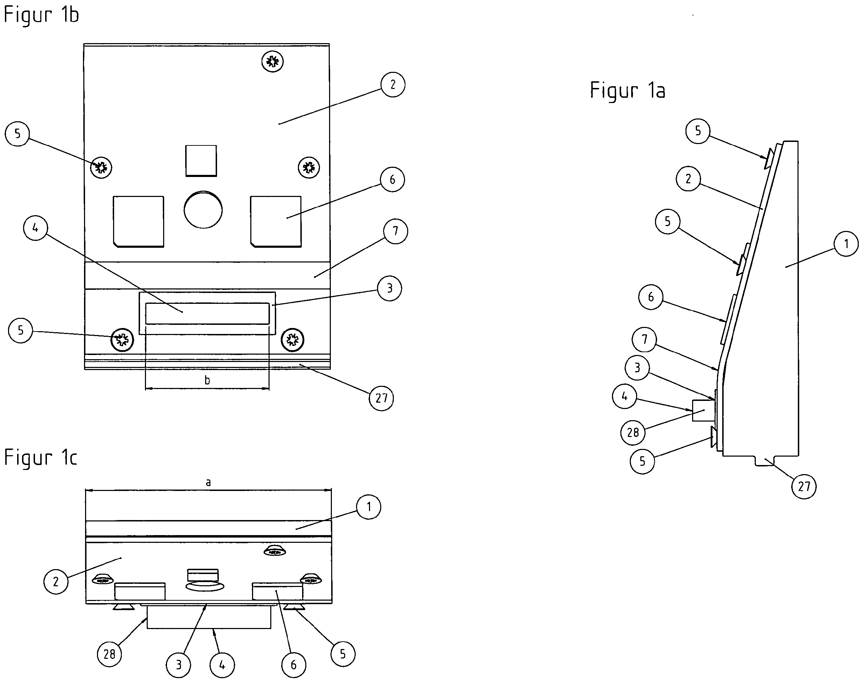

Figur 1 setzt sich aus drei Figuren 1a, 1b und 1c zusammen, die dasselbe Bauteil - ein Trägermodul 1 - aus verschiedenen Blickrichtungen beziehungsweise in einem Querschnitt (Figur 1a) darstellen. Figur 1b zeigt die Ansicht des Trägermoduls 1 von links auf Figur 1a und Figur 1c zeigt das Trägermodul 1 aus der Blickrichtung von oben auf Figur 1a.

In Figur 1a ist zu erkennen, dass der Querschnitt des Trägermoduls 1 im Wesentlichen trapezförmig ist, wobei die obere rechte Spitze des Trapezes abgeschnitten ist. Diese Spitze wird durch die hintere, vertikal verlaufende Fläche und die schräg verlaufende Fläche, auf der der mit elektronischen Bauteilen 6 bestückte, abgeknickte Teil einer Leiterplatine 2 angeordnet ist, gebildet. Auf der in Figur 1a links dargestellten Oberfläche des Trägermoduls 1 ist die Leiterplatine 2 über Befestigungsschrauben 5 am Trägermodul 1 festgelegt. Auf der Leiterplatine 2 ist in einem in Figur 1a vertikal dargestellten unteren Bereich eine Photodiode 3 aufgebracht. Die Photodiode 3 weist einzelne Pixel auf, die jeweils gleiche aktive Bereiche hat und zwischen denen jeweils äquidistante Abstände gegeben sind.

Die Leiterplatine 2 erstreckt sich nach oben über einen Knickbereich 7 in einen schräg verlaufenden oberen Bereich. In diesem oberen Bereich sind verschiedene elektronische Bauteile 6 angeordnet, wie beispielsweise ein Multiplexer und ein A/D-Wandler. Dadurch ist es möglich, dass die von der Photodiode 3 detektierten Lichtimpulse ohne zwischengeschaltete lange Leitungen direkt auf der Leiterplatine 2 vorverarbeitet und in digitale Signale umgewandelt werden können. Dadurch wird einem Qualitätsverlust aufgrund langer analoger Leitungen und Schnittstellen, wie Steckverbindungen, vorgebeugt.

Auf die Photodiode 3 ist ein Szintillatorelement 4 aufgeklebt. Dieses erstreckt sich in der Horizontalen über eine Länge b (siehe Figur 1b), die genau so groß ist, dass der aktive Bereich der Photodiode 3, also vom linken Rand des linken Randpixels bis zum rechten Rand des rechten Randpixels, und zusätzlich noch ein Zwischenraum zwischen zwei benachbarten Pixeln innerhalb der Photodiode 3 überdeckt wird. Wie aus Figur 1b ersichtlich, erstreckt sich die Photodiode 3 am Rand noch wegen fertigungstechnischer Notwendigkeiten über die Länge b des Szintillatorelements 4 hinaus. In diesem überschießenden Teil ist jedoch keine aktive Fläche, also keine Pixel der Photodiode 3 mehr enthalten. Das Szintillatorelement 4 dient dazu, von unten in Figur 1a (gegebenenfalls auch von oben - dazu weiter unten noch mehr) einfallende Röntgenstrahlung in Lichtquanten umzuwandeln, die dann schräg beziehungsweise vertikal auf die Photodiode 3 treffen und dort detektiert werden.

An der in Figur 1a dargestellten unteren Fläche des Trägermoduls 1 ist eine Ausnehmung 27 ausgebildet, die sich über die gesamte Breite a (siehe Figur 1c) des Trägermoduls 1 erstreckt.

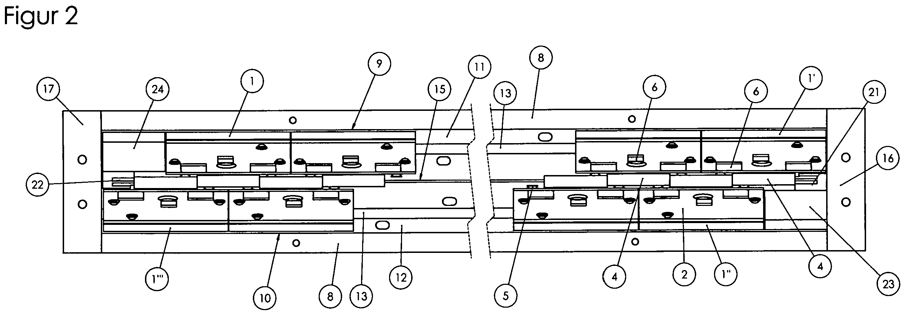

Die Breite a des Trägermoduls 1 beziehungsweise der darauf angebrachten Leiterplatine 2 ist ungefähr doppelt so groß wie die Länge b des Szintillatorelements 4. Erfindungsgemäß ist es notwendig, dass diese Breite a höchstens das Doppelte der Länge b des Szintillatorelements 4 aufweist, so dass die in Figur 2 dargestellte Anordnung aller Trägermodule 1 in einem Röntgenzeilendetektor realisiert werden kann. Gleichzeitig ist es jedoch vorteilhaft, wenn diese Breite a nahe an das doppelte der Länge b des Szintillatorelements 4 herankommt, um möglichst alle elektronischen Bauteile 6 auf der Leiterplatine 2 unterbringen zu können, die nötig sind, um die in der Photodiode 3 empfangenen Signale dort vorzuverarbeiten und zu digitalisieren.

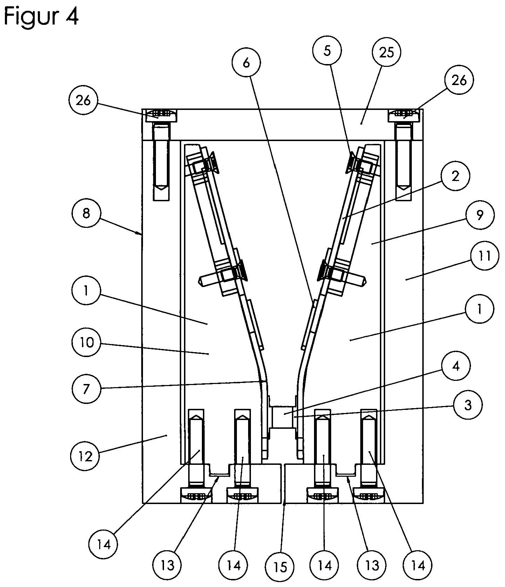

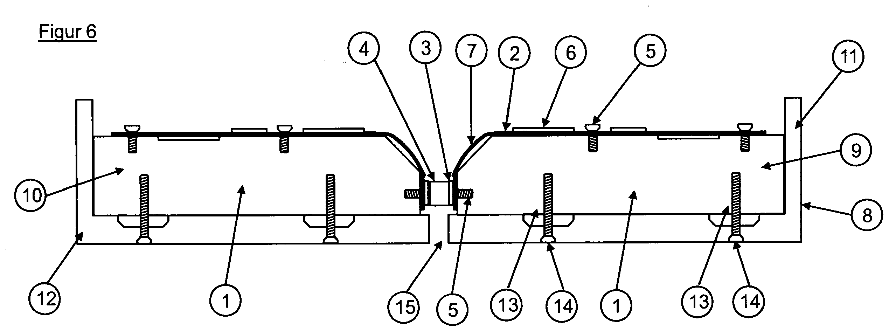

Der Winkel im Trägermodul 1 innerhalb des Knickbereichs 7 der Leiterplatine 2 kann erfindungsgemäß über einen weiten Bereich variieren. Beispielsweise kann dieser auch 90° betragen, wie dies im zweiten Ausführungsbeispiel der Figur 6 dargestellt ist. Es ist allerdings auch durchaus möglich, dass dieser Winkel 0° beträgt, so dass eine ebene Leiterplatine 2 verwendet werden kann. In diesem Fall ist der Querschnitt des Trägermoduls 1 rechteckig. Auf die Vorteile einer geknickten Leiterplatine 2 wird unten bei der Beschreibung zur Figur 4 näher eingegangen.

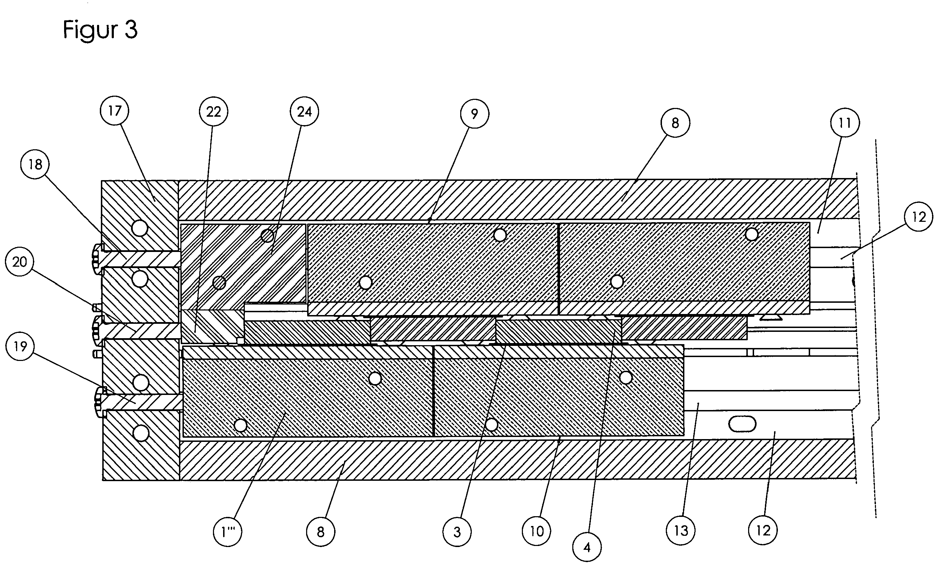

In Figur 2 ist ein gesamter, fertig montierter Röntgenzeilendetektor mit einer Vielzahl von Trägermodulen 1 aus einer Blickrichtung gemäß Figur 1c, also in Bezug auf Figur 1a von oben gesehen, dargestellt. In Figur 3 ist die Anordnung in einem Längsschnitt in einer parallel zu der Zeichenebene der Figur 2 verlaufenden Ebene dargestellt, wobei im linken Bereich drei Spannschrauben 18, 19, 20 innerhalb von Durchbrechungen in einer zweiten Abschlussplatte 17 dargestellt sind, die durch die zweitgenannte hindurch greifen und deshalb in Figur 2 nicht sichtbar sind. Die Figuren 2 und 3 werden im Weiteren zusammen beschrieben, wobei immer nur explizit auf Figur 2 hingewiesen wird, es sei denn Merkmale sind nur in Figur 3 zu erkennen - dann ist dies ausgeführt.

Der fertig montierte Röntgenzeilendetektor - der in seinem Querschnitt aus derselben Richtung wie Figur 1a betrachtet, in Figur 4 dargestellt ist - weist ein Gehäuse 8 auf, das zur Aufnahme der einzelnen Trägermodule 1 dient. Im Folgenden werden die Figuren 2 bis 4 zusammen beschrieben, ohne dass jeweils im Regelfall darauf eingegangen wird, in welcher der Figuren das gerade beschriebene Merkmal zu erkennen ist.

Das Gehäuse 8 weist ein erstes Trägerelement 11 und ein zweites Trägerelement 12 auf, die im Querschnitt im Wesentlichen jeweils L-förmig ausgebildet sind und spiegelsymmetrisch zu einer in Figur 2 horizontal ausgerichteten und senkrecht auf der Blattebene stehenden Symmetrieebene angeordnet sind. In den in Figur 4 jeweils horizontal verlaufenden Schenkeln der beiden Trägerelemente 11, 12 ist jeweils eine Nut 13 ausgebildet, die sich über die gesamte Länge (in Figur 4 senkrecht zur Blattebene) erstreckt. Diese Nut 13 ist komplementär zu den Nasen 27 (siehe Figur 1a) der Trägermodule 1 ausgebildet, so dass in Figur 4 in vertikaler und horizontaler Richtung innerhalb der Blattebene ein Formschluss zwischen diesen Nasen 27 und den Nuten 13 gegeben ist. Ein Trägermodul 1, das somit in eine dieser Nuten 13 gesetzt wird, hat dadurch nur noch einen Freiheitsgrad senkrecht zur Blattebene, also entlang der Länge des Röntgenzeilendetektors (in Figur 2 ist dies die Horizontale).

An den beiden Stirnseiten des Gehäuses 8 sind eine erste Abschlussplatte 16 (rechts in Figur 2) und eine zweite Abschlussplatte 17 (links in Figur 2) mit den beiden Trägerelementen 11, 12 verbunden. Bevorzugt handelt es sich hierbei um lösbare Schraubverbindungen. Die Dimensionen der beiden Trägerelemente 11, 12 und der beiden Abschlussplatten 16, 17 zueinander ist so gewählt, dass zwischen den beiden horizontal verlaufenden Schenkeln in Figur 4 der beiden Trägerelemente 11, 12 ein Eintrittsschlitz 15 ausgebildet ist. Dieser Eintrittsschlitz 15 erstreckt sich über die gesamte Länge des Gehäuses 8 von der ersten Abschlussplatte 16 bis zur zweiten Abschlussplatte 17. Die Szintillatorelemente 4 der einzelnen Trägermodule 1 sind dabei so ausgerichtet, dass sie in der Verlängerung des Eintrittsschlitzes 15 in Figur 4 nach oben angeordnet sind.

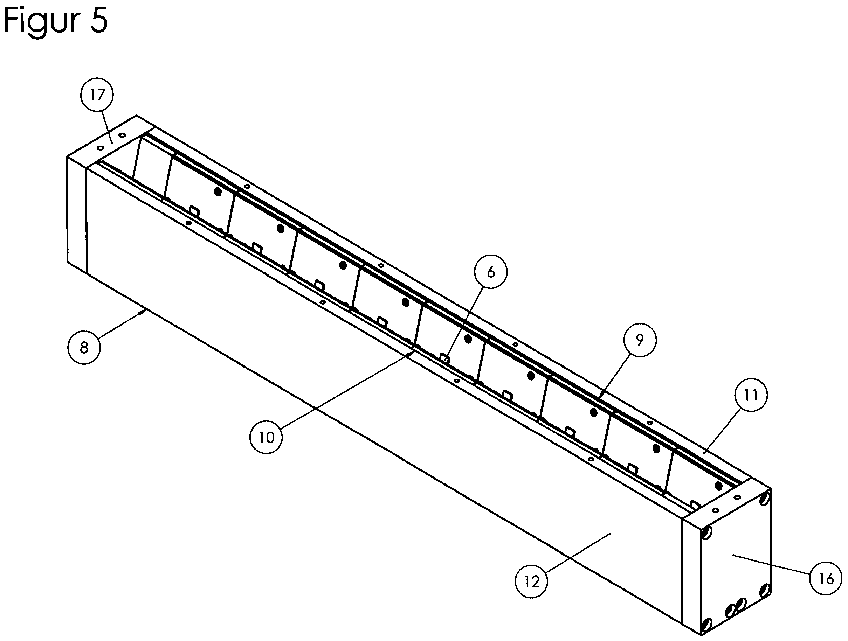

Zur mechanischen Stabilisierung des gesamten Gehäuses 8 und darüber hinaus zur mechanischen und optischen Abschottung gegen störende Umwelteinflüsse von oben ist an den in Figur 4 vertikal dargestellten Schenkeln der L-förmigen Trägerelemente 11, 12 eine Deckelplatte 25 mittels Deckelschrauben 26 verbunden; andere Verbindungsmöglichkeiten sind genauso möglich. In Figur 2 ist zur besseren Erkennbarkeit der darunter liegenden Trägermodule 1 die Deckelplatte 25 nicht dargestellt. Dasselbe gilt auch für die schräg von oben dargestellte perspektivische Ansicht des fertigen Röntgenzeilendetektors der Figur 5, die nur zur besseren räumlichen Vorstellung des gesamten Röntgenzeilendetektors in Verbindung mit den Figuren 2 bis 4 hinzugefügt ist.

Innerhalb des Gehäuses 8 ist auf dem ersten Trägerelement 11 eine erste Reihe 9 von Trägermodulen 1 aufgesetzt. Auf dem gegenüber liegenden zweiten Trägerelement 12 ist eine zweite Reihe 10 von Trägermodulen 1 aufgesetzt. Die Trägermodule 1 sind alle gleich ausgebildet, wobei sich Ihre Ausrichtung auf den beiden Trägerelementen 11 und 12 so gestaltet, dass sie jeweils mit ihrem Szintillatorelement 4 in Richtung auf die andere Reihe 9, 10 von Trägermodulen 1 ausgerichtet sind. Die Anordnung der Trägermodule 1 ist dabei so, dass ihre Szintillatorelemente 4 an deren jeweiliger Stirnseite 28 aneinanderstoßen. Dies bedeutet, dass immer ein Trägermodul der ersten Reihe 9 durch die beiden versetzt zu ihm angeordneten Trägermodule 1 der zweiten Reihe 10 Szintillatorelement 4 an Szintillatorelement 4 angeordnet sind. Dasselbe gilt für jedes der Trägermodule 1 der zweiten Reihe 10 in umgekehrter Beziehung hinsichtlich der Trägermodule 1 der ersten Reihe 9. Es versteht sich von selbst, dass dies nicht für die den beiden Abschlussplatten 16, 17 jeweils am nächsten liegenden Trägerelemente 1', 1''' gilt, da diese nur einen schräg dazu versetzten Partner auf der jeweils anderen Reihe 9, 10 aufweisen (siehe Figur 2).

Da die Breite a der Trägermodule 1 und der darauf angeordneten Leiterplatinen 2 nicht ganz doppelt so groß ist wie die Länge b der Szintillatorelemente 4, stoßen die Trägermodule 1 in jeder Reihe 9, 10 nicht aneinander an. Zwischen diesen ist jeweils ein kleiner Zwischenraum ausgebildet. Wie aus Figur 2 gut erkennbar ist, sind die Photodioden 3 in ihren über die Szintillatorelemente 4 hinaus stehenden Teilen überlappend gegenüber den auf der jeweils anderen Reihe 9, 10 befindlichen Photodioden 3. Da jedoch die Szintillatorelemente 4 genau die Breite der aktiven Flächen der Photodioden 3, also der Pixel und zusätzlich noch den Zwischenraum zwischen zwei benachbarten Pixeln einer Photodiode 3 überdecken, werden somit die im Stand der Technik vorhandenen Probleme, insbesondere einer Verzerrung im Bereich zwischen zwei Photodioden 3, vermieden.

An der ersten Abschlussplatte 16 stößt ein erstes Distanzstück 21 an, das in der Verlängerung der Stirnflächen 28 der Szintillatorelemente 4 ausgerichtet ist und aus einem Material geringer Elastizität besteht. An diesem setzt dann mit seiner einen Stirnfläche 28 das Szintillatorelement 4 des ersten Trägermoduls 1' an. Bevorzugt wird die Länge des ersten Distanzstücks 21 so gewählt, dass noch ein geringer Abstand zwischen dem ersten Trägermodul 1' und der ersten Abschlussplatte 16 besteht, damit bei der Montage - die unten noch näher beschrieben wird - eine leichte Deformation durch Zusammendrücken des ersten Distanzstücks 21 in horizontaler Richtung in Figur 2 ermöglicht wird und das erste Trägermodul 1' dann an der ersten Abschlussplatte 16 anschlägt.

Zwischen der ersten Abschlussplatte 16 und dem zweiten Trägermodul 1'', das mit seinem Szintillatorelement 4 links an das Szintillatorelement 4 des ersten Trägermoduls 1' (siehe Figur 2) anschlägt, ist ein erstes Pufferstück 23 angeordnet. Dieses weist eine Länge in horizontaler Richtung in Figur 2 auf, die ungefähr die Hälfte der Breite a eines Trägermoduls 1 beträgt, also ungefähr gleich groß ist wie die Länge b eines Szintillatorelements 4. Auch dieses erste Pufferstück 23 ist aus einem leicht elastischen Material, wie zum Beispiel Hartgummi (vergleichbar dem ersten Distanzstück 21). Alternativ kann das erste Pufferstück 23 etwas schmaler und aus dem gleichen Material wie die Trägermodule 1, regelmäßig einem Metall, sein. Dann sorgt ein dünner Gummipuffer für das seitliche Spiel.

Am linken Ende des Röntgenzeilendetektors in Figur 2 ist ein zweites Distanzstück 22 in der Verlängerung der Szintillatorelemente 4 angeordnet, welches im Wesentlichen gleich ist, wie das erste Distanzstück 21. Außerdem ist auf der dem letzten Trägermodul 1''' entgegengesetzen Trägerelement 11 ein zweites Pufferstück 24 angeordnet, das im Wesentlichen gleich ausgebildet ist, wie das erste Pufferstück 23.

In Figur 3 ist zu erkennen, dass auf das zweite Pufferstück 24 eine erste Spannschraube 18 wirkt, die durch ein Schraubloch in der zweiten Abschlussplatte 17 in horizontaler Richtung in Figur 3 auf die erste Reihe 9 hin oder von dieser weg gedreht werden kann. Zwischen dem letzen Trägermodul 1*'''* der zweiten Reihe 10 und der zweiten Abschlussplatte 17 ist eine zweite Spannschraube 19 angeordnet, die ebenfalls durch ein Schraubloch in der zweiten Abschlussplatte 17 auf die zweite Reihe 10 hin oder von dieser weg gedreht werden kann. Schließlich ist noch eine dritte Spannschraube 20 in einem weiteren Schraubloch der zweiten Abschlussplatte 17 vorhanden, die in horizontaler Richtung in Figur 3 auf das zweite Distanzstück 22 und somit auf die Reihe von Szintillatorelementen 4 durch Einschrauben Druck ausüben kann beziehungsweise durch Herausschrauben Druck von diesen nehmen kann.

Im Folgenden wird erläutert, wie nach einem erfindungsgemäßen Verfahren ein oben näher beschriebener erfindungsgemäßer Röntgenzeilendetektor zusammengebaut werden kann.

Zuerst wird das erste Distanzstück 21 in das Gehäuse 8 eingesetzt. Danach wird das erste Trägermodul 1' mit seiner Ausnehmung 27 so auf die Schiene 13 des ersten Trägerelements 11 gesetzt, dass es mit seinem Szintillatorelement 4 in Richtung auf das zweite Trägerelement 12 weist. Es wird so weit in Richtung der ersten Abschlussplatte 16 geschoben, bis die Stirnseite 28 seines Szintillatorelements 4 an dem ersten Distanzstück 21 anschlägt. Es wird ein erstes Pufferstück 23 auf das zweite Trägerelement 12 gesetzt und in Anschlag an die erste Abschlussplatte 16 gebracht. Danach wird das zweite Trägermodul 1" so auf das zweite Trägerelement 12 gesetzt, dass sein Szintillatorelement 4 in Richtung auf das erste Trägerelement 11 weist. Das zweite Trägermodul 1" wird entlang der Schiene 13 soweit auf die erste Abschlussplatte 16 zu verschoben, bis die Stirnfläche 28 seines Szintillatorelements 4 an der Stirnfläche 28 des Szintillatorelements 4 des ersten Trägermoduls 1' anschlägt. Dabei ist regelmäßig auch ein Kontakt des zweiten Trägermoduls 1'' mit dem ersten Pufferstück 23 gegeben.

Die im vorherigen Absatz angegebene Reihenfolge kann auch so geändert werden, dass nach dem Einsetzen des ersten Distanzstücks 21 zuerst das erste Pufferstück 23 eingesetzt wird, bevor dann das erste Trägermodul 1' und danach da zweite Trägermodul 1" eingesetzt und jeweils wie oben beschrieben verschoben werden. Alternativ kann die Reihenfolge auch mit dem Einsetzen des ersten Pufferstücks 23 und anschließendem Einsetzen des ersten Distanzstücks 21 beginnen und danach das erste Trägermodul 1' und anschließend das zweite Trägermodul 1" eingesetzt und jeweils wie oben ausgeführt verschoben werden.

Anschließend wird auf das erste Trägerelement 11 ein weiteres Trägermodul 1 mit der gleichen Ausrichtung wie das erste Trägermodul 1' aufgesetzt und soweit in Richtung der ersten Abschlussplatte 16 verschoben, bis die Stirnfläche 28 seines Szintillatorelements 4 an der Stirnfläche 28 des Szintillatorelements 4 des zweiten Trägermoduls 1" anschlägt.

Es werden dann immer abwechselnd weitere Trägermodule auf das zweite Trägerelement 12 beziehungsweise erste Trägerelement 11 gesetzt, wobei die Szintillatorelemente 4 jeweils in Richtung des gegenüber liegenden Trägermoduls 1 weisen und dann soweit wie möglich - also bis zum Anschlag am nächsten Szintillatorelement 4 mit dem eigenen Szintillatorelement 4 auf die erste Abschlussplatte 16 hin verschoben werden.

Nachdem das letzte Trägermodul 1''' auf dem zweiten Trägerelement 12 - dies ist nicht zwingend, es könnte auch auf dem ersten Trägerelement 11 aufgesetzt werden - aufgesetzt wurde, wird ein zweites Pufferstück 24 zwischen der zweiten Abschlussplatte 17 und dem letzten, sich auf dem ersten Trägerelement 11 befindenden Trägermodul 1 auf das erste Trägerelement 11 aufgesetzt. Außerdem wird auch noch ein zweites Distanzstück 22 im Anschluss an die Stirnfläche 28 des letzten Trägermoduls 1''' zwischen diesem und der zweiten Abschlussplatte 17 angeordnet.

Danach wird eine Grobjustierung der Trägermodule 1 durch abwechselndes Eindrehen der ersten Spannschraube 18 und der zweiten Spannschraube 19 in Richtung auf die erste Abschlussplatte 16, bis das letzte Trägermodul 1''' an einer vordefinierten Stelle zu liegen kommt. Dies wird durch die Übereinstimmung einer Markierung am Gehäuse 8 mit einer Markierung am letzten Trägermodul 1''' erreicht. Im Anschluss daran erfolgt die Feinjustierung, indem die dritte Spannschraube 20 so weit angezogen wird, also in Richtung auf die erste Abschlussplatte 16 hin eingedreht wird, bis die beiden oben genannten Markierungen am letzten Trägermodul 1''' und am Gehäuse exakt übereinstimmen, wobei sämtliche Szintillatorelemente 4 mit ihren jeweils gegenüber liegenden Stirnflächen 28 gegeneinander gepresst sind.

Um zu verhindern, dass die unter Druck gegeneinander gepressten Szintillatorelemente 4 abbrechen - dies gilt insbesondere, wenn diese schon über eine gewisse Zeit Röntgenstrahlung ausgesetzt waren und somit der Kleber zwischen ihnen und den darunter liegenden Photodioden 3 an Qualität verloren hat - werden diese wieder entlastet. Dies ist insbesondere auch bei Transport der Röntgendetektorzeile vorteilhaft. Zur Entlastung werden die einzelnen Trägermodule 1 durch mindestens jeweils eine zugeordnete Festlegeschraube 14 an dem jeweiligen Trägerelement 11, 12 festgelegt. Dafür weist das jeweilige Trägerelement 11, 12 an seiner Unterseite in Figur 4 ein Langloch auf, durch das die jeweilige Festlegeschraube 14 in ein Schraubloch an der Unterseite des Trägermoduls 1 eingeschraubt werden kann. Da die einzelnen Trägermodule 1 nun vollständig fixiert sind an dem jeweiligen Trägerelement 11, 12 und somit die Äquidistanz zwischen den einzelnen Pixeln der Photodioden 3 - auch im Bereich der aneinander angrenzenden, sich gegenüberliegenden Photodioden 3 - gewährleistet ist, kann die dritte Spannschraube 20 wieder gelöst werden und somit der Druck auf die Szintillatorelemente 4 von diesen genommen werden. Dadurch wird ein Abbrechen derselben von den Photodioden 3 verhindert beziehungsweise das Risiko hierzu stark vermindert.

Auch die beiden anderen Spannschrauben 18, 19 können wieder gelockert werden, da keine Verschiebung der einzelnen Trägermodule 1 mehr befürchtet werden muss, da diese an den Trägerelementen 11, 12 festgelegt sind.

Zum Abschluss wird dann auf der oben noch offenen Seite (siehe Figur 4) des Gehäuses 8 die Deckelplatte 25 mit dafür vorgesehenen Deckelschrauben 26 befestigt. Somit erhält man eine bis auf den Eintrittsschlitz 15 an der Unterseite des Röntgenzeilendetektors (wie in Figur 4 dargestellt) in sich geschlossene Einheit. Der Schlitz selbst wird mit einer dünnen Metall-Folie gegen Umwelteinflüsse abgeschottet.

Die Deckelplatte 25 ist aus einem Material, das nur eine geringe Menge an (rückgestreuter) Röntgenstrahlung erzeugt, wie beispielsweise Carbon.

Die Trägermodule 1 und die darauf befindlichen Leiterplatinen 2 werden mit einem Knickbereich 7 versehen, damit die direkt einfallende Röntgenstrahlung (in Figur 4 im Normalfall von unten durch den Eintrittsschlitz 15 eintretend) beziehungsweise die an den Szintillatorelementen 4 gestreute Röntgenstrahlung nicht die sehr empfindlichen elektronischen Bauteile 6 auf dem oberen Teil der Leiterplatine 2 treffen, um Störungen zu vermeiden. Dies wird dadurch erreicht, dass der Grundkörper des Trägermoduls 1 als Abschirmung für die elektronischen Bauteile 6 fungiert, da egal von welchem Punkt des Szintillatorelements 4 ausgehende Streustrahlung niemals direkt auf die elektronischen Bauteile 6 auftreffen kann. Sie wird durch den massiven Körper des Trägermoduls 1 absorbiert.

In diesem Zusammenhang sei darauf hingewiesen, dass der Röntgenzeilendetektor auch in "umgekehrter Richtung" eingesetzt werden kann. Dies bedeutet also, dass die Röntgenstrahlung nicht von unten in Figur 4 durch den Eintrittsschlitz 15 eintritt und innerhalb der Szintillatorelemente 4 die dann in den Photoelektroden 3 detektierte Lichtquanten erzeugt, sondern die Röntgenstrahlung in Figur 4 von oben durch die Deckelplatte 25 einfällt. Dies wird insbesondere bei höherenergetischer Röntgenstrahlung in einem Bereich von über 1,5 MeV verwendet. Um auch bei dieser Ausrichtung zu gewährleisten, dass möglichst keine Streustrahlung die empfindlichen elektronischen Bauteile 6 trifft, muss zum einen die Deckelplatte 25 aus einem Material bestehen, dass nur sehr geringe Menge an Streustrahlung erzeugt, wie beispielsweise Carbon. Zum anderen muss außerhalb des Röntgenzeilendetektors eine Abschirmung für die ansonsten direkt auf die elektronischen Bauteile 6 treffenden Röntgenstrahlung vorhanden sein. Diese Abschirmung (nicht dargestellt) kann auch gleichzeitig den Eintrittsschlitz definieren, der geometrisch sehr genau auf die Szintillatorelemente 4 und den Schlitz 15 ausgerichtet sein muss, damit der Röntgenzeilendetektor korrekt arbeitet. Aufgrund der sehr hohen Masse dieser Vorrichtung wird sie ortsfest vom Anwender als separates Bauteil zur Verfügung gestellt. Der Vorteil dieser Strahlrichtung ist, dass der größte Teil der Strahlung den Detektor ungestört durchdringt und hinter dem Detektor hoch-energetische Streustrahlung erzeugt; zum Schutz gegen diese Streustrahlung ist die "serienmäßige" Abschirmung ausreichend dimensioniert.

In Figur 6 ist ein zweites Ausführungsbeispiel eines erfindungsgemäßen Röntgenzeilendetektors dargestellt, dessen einziger grundlegender Unterschied zu dem vorher beschriebenen ersten Ausführungsbeispiel darin liegt, dass der Knickbereich 7 einen Winkel von 90° aufweist. Dadurch ist es möglich, dass der gesamte Röntgenzeilendetektor flacher baut, wobei er dadurch logischerweise aber breiter (in der Horizontalen in Figur 6) wird. Die Funktionsweise ist genauso wie zum ersten Ausführungsbeispiel beschrieben, so dass hier darauf verzichtet wird, dies zu wiederholen. Ein Unterschied besteht insbesondere darin, dass aufgrund der größeren Auflagefläche im unteren Bereich der Trägermodule 1 auf den horizontalen Schenkeln der Trägerelemente 11, 12 jeweils zwei Nasen 27 pro Trägermodul 1 ausgebildet sind, die dann in diesen zugeordneten zwei Nuten 13 an den Trägerelementen 11, 12 aufgesetzt werden. Zur besseren Fixierung der Trägermodule 1 an den Trägerelementen 11, 12 werden dann für jede der beiden Ausnehmungen 27 Festlegeschrauben 14 verwendet.

Die Ansicht der Figur 6 entspricht im Wesentlichen derjenigen der Figur 4, wobei noch keine Deckelplatte 25 am oberen Ende der Trägerelemente 11, 12 angebracht ist.

### Bezugszeichenliste

- 1: Trägermodul
- 1': erstes Trägermodul
- 1": zweites Trägermodul
- 1''': letztes Trägermodul
- 2: Leiterplatine
- 3: Photodiode
- 4: Szintillatorelement
- 5: Befestigungsschraube
- 6: elektronisches Bauteil
- 7: Knickbereich
- 8: Gehäuse
- 9: erste Reihe
- 10: zweite Reihe
- 11: erstes Trägerelement
- 12: zweites Trägerelement
- 13: Nut
- 14: Festlegeschraube
- 15: Eintrittsschlitz
- 16: erste Abschlussplatte
- 17: zweite Abschlussplatte
- 18: erste Spannschraube
- 19: zweite Spannschraube
- 20: dritte Spannschraube
- 21: erstes Distanzstück
- 22: zweites Distanzstück
- 23: erstes Pufferstück
- 24: zweites Pufferstück
- 25: Deckelplatte
- 26: Deckelschraube
- 27: Nase
- 28: Stirnfläche
- a: Breite des Trägermoduls
- b: Länge des Szintillatorelements

## Patentansprüche

1. Röntgenzeilendetektor mit einer vorgegebenen Anzahl von Trägermodulen (1), die bis auf eine Fertigungstoleranz die gleiche Breite (a) aufweisen und in einem Gehäuse (8) angeordnet sind,
wobei auf jedem Trägermodul (1) eine einstückige Leiterplatine (2), auf der eine Photodiode (3) angeordnet ist, angebracht ist,
wobei diese Leiterplatine (2) breiter ist als die aktive Fläche der die Photodiode (3) bildenden Pixel,
wobei auf jeder Photodiode (3) jeweils ein Szintillatorelement (4) aufgebracht ist, das in seiner Länge (b) genau die aktive Fläche in ihrer Breite zuzüglich eines Zwischenraums zwischen zwei benachbarten Pixeln einer Photodiode (3) bedeckt,
wobei die Breite (a) jedes Trägermoduls (1) höchstens doppelt so groß ist wie die Länge (b) eines Szintillatorelements (4),
wobei die Trägermodule (1) in zwei Reihen (9, 10) so im Gehäuse (8) angeordnet sind, dass sich die Photodioden (3) jeder Reihe (9, 10) gegenüberliegen,
wobei die Szintillatorelemente (4) auf Anschlag an ihren jeweiligen Stirnseiten (28) aneinanderstoßen,
wobei aneinander anschlagende Szintillatorelemente (4) in jeweils gegenüberliegenden Reihen (9, 10) angeordnet sind, so dass jeweils ein Trägermodul (1) der einen Reihe (9; 10) durch die beiden versetzt zu ihm angeordneten Trägermodule (1) der anderen Reihe (10; 9) dazu führt, dass Szintillatorelement (4) an Szintillatorelement (4) angeordnet ist.

2. Röntgenzeilendetektor nach Patentanspruch 1, **dadurch gekennzeichnet, dass** das Gehäuse (8) ein erstes Trägerelement (11) und ein dazu spiegelsymmetrisch angeordnetes und ausgebildetes zweites Trägerelement (12) aufweist, zwischen denen ein Eintrittsschlitz (15) für Röntgenstrahlung ausgebildet ist, wobei die Trägermodule (1) auf diesen beiden Trägerelementen (11, 12) alternierend angeordnet sind und die Szintillatorelemente (4) in der Verlängerung zum Eintrittsschlitz (15) angeordnet sind.

3. Röntgenzeilendetektor nach Patentanspruch 2, **dadurch gekennzeichnet, dass** die Trägerelemente (11, 12) L-förmig ausbildet sind und in jedem Trägerelement (11, 12) jeweils eine parallel zum Eintrittsschlitz (15) ausgerichtete Nut (13) ausgebildet ist, in die formschlüssig eine an den Trägermodulen (1) ausgebildete Nase (27) eingreift.

4. Röntgenzeilendetektor nach einem der vorstehenden Patentansprüche, **dadurch gekennzeichnet, dass** jedes Trägermodul (1) über mindestens ein Festlegeelement, insbesondere eine Festlegeschraube (14), mit dem jeweiligen Trägerelement (11, 12) verbunden ist.

5. Röntgenzeilendetektor nach einem der Patentansprüche 2 bis 4, **dadurch gekennzeichnet, dass** an den Enden des Gehäuses (8) jeweils eine Abschlussplatte (16, 17) angeordnet ist, wobei die erste Abschlussplatte (16) als ein Anschlag und die zweite Abschlussplatte (17) als ein Spannelement ausgebildet sind.

6. Röntgenzeilendetektor nach Patentanspruch 5, **dadurch gekennzeichnet, dass** die zweite Abschlussplatte (17) drei Spannschrauben (18, 19, 20) aufweist, von denen die erste Spannschraube (18) mit der ersten Reihe (9) von Trägermodulen (1), die zweite Spannschraube (19) mit der zweiten Reihe (10) von Trägermodulen (1) und die dritte Spannschraube (20) mit den Szintillatorelementen (4) zusammenwirken.

7. Röntgenzeilendetektor nach einem der Patentansprüche 2 bis 6, **dadurch gekennzeichnet, dass** das Gehäuse (8) an seiner dem Eintrittsschlitz (15) gegenüberliegenden Seite eine Deckelplatte (25) aufweist.

8. Röntgenzeilendetektor nach Patentanspruch 7, **dadurch gekennzeichnet, dass** die Deckelplatte (25) aus einem Material ist, das bei der verwendeten Energie der Röntgenstrahlung kaum Streustrahlung produziert, insbesondere ist sie aus Carbon.

9. Röntgenzeilendetektor nach einem der Patentansprüche 5 bis 8, **dadurch gekennzeichnet, dass** zwischen der Abschlussplatte (16) und einer der beiden Reihen (9, 10) von Trägermodulen (1) ein erstes Pufferstück (23) eingesetzt ist, das halb so breit wie die Trägermodule (1) ist, und zwischen der ersten Abschlussplatte (16) und den Szintillatorelementen (4) ein erstes Distanzstück (21) eingesetzt ist, das halb so breit wie die Szintillatorelemente (4) ist.

10. Röntgenzeilendetektor nach Patentanspruch 9, **dadurch gekennzeichnet, dass** zwischen der Abschlussplatte (17) und einer der beiden Reihen (9, 10) von Trägermodulen (1) ein zweites Pufferstück (24) eingesetzt ist, das halb so breit wie die Trägermodule (1) ist, und zwischen der zweiten Abschlussplatte (17) und den Szintillatorelementen (4) ein zweites Distanzstück (22) eingesetzt ist, das halb so breit wie die Szintillatorelemente (4) ist.

11. Röntgenzeilendetektor nach einem der vorstehenden Patentansprüche, **dadurch gekennzeichnet, dass** die Leiterplatinen (2) abgeknickt sind und in dem Bereich, der auf der anderen Seite des Knickbereichs (7) in Bezug auf die Photodiode (3) ausgebildet ist, elektronische Bauteile (6) angeordnet sind.

12. Verfahren zur Herstellung eines Röntgenzeilendetektors mit den Merkmalen eines der vorangehenden Patentansprüche mit folgenden Schritten:
- Einsetzen eines ersten Distanzstücks (21) an einer ersten Abschlussplatte (16) und nachfolgendes Einsetzen eines ersten Trägermoduls (1') an einem ersten Trägerelement (11) bis das Szintillatorelement (4) des ersten Trägermoduls (1') am ersten Distanzstück (21) anschlägt und Einsetzen eines ersten Pufferstücks (23) an einem zweiten Trägerelement (12) bis es an der ersten Abschlussplatte (16) anschlägt, wobei das Einsetzen des ersten Trägermoduls (1') und des ersten Pufferstücks (23) auch in umgekehrter Reihenfolge erfolgen kann;
- alternativ zum vorgenannten Schritt kann auch zuerst das erste Pufferstück (23) am zweiten Trägerelement (12) auf Anschlag an die erste Abschlussplatte (16) und danach das erste Distanzstück (21) auf Anschlag an die erste Abschlussplatte (16) gebracht werden und anschließend wird das erste Trägermodul (1') mit seinem Szintillatorelement (4) auf Anschlag an das erste Distanzstück (21) gebracht;
- nach einem der beiden zuvor genannten alternativen Schritte wird ein zweites Trägermodul (1") so eingesetzt, dass sein Szintillatorelement (4) am Szintillatorelement (4) des ersten Trägermoduls (1') und es selbst am ersten Pufferstück (23) anschlägt;
- anschließend wird im Wechsel immer ein Trägermodul (1) am ersten und am zweiten Trägerelement (11, 12) eingesetzt, wobei deren jeweiliges Szintillatorelement (4) am Szintillatorelement (4) seines direkt zuvor eingesetzten Trägermoduls (1) anschlägt;
- in beliebiger Reihenfolge wird ein zweites Distanzstück (22), das halb so breit wie ein Szintillatorelement (4) ist, an das Szintillatorelement (4) des letzten Trägermoduls (1''') auf Anschlag gesetzt und ein zweites Pufferstück (24), das halb so breit wie ein Trägermodul (1) ist, wird an das als vorletztes eingesetzte Trägermodul (1) auf Anschlag gesetzt;
- Spannmittel werden in einer zweiten Abschlussplatte (17), insbesondere eine dritte Spannschraube (20), so betätigt, dass alle Szintillatorelemente (4) mit ihren sich jeweils gegenüberliegenden Stirnflächen (28) aneinandergepresst werden;
- Festlegen der beiden Reihen (9, 10) von Trägermodulen (1) mit Spannmitteln in der zweiten Abschlussplatte (17), insbesondere mittels einer ersten und einer zweiten Spannschraube (18, 19).

13. Verfahren nach Patentanspruch 12, **dadurch gekennzeichnet, dass** im Anschluss die einzelnen Trägermodule (1) mittels Festlegemitteln, insbesondere mit Festlegeschrauben (14), am Gehäuse (8) festgelegt werden und danach die dritte Spannschraube (20) gelockert wird, so dass auf die Szintillatorelemente (4) kein Druck mehr wirkt.

14. Verfahren nach Patentanspruch 12 oder 13, **dadurch gekennzeichnet, dass** anschließend eine Deckelplatte (25) mit dem Gehäuse (8) verbunden, insbesondere verschraubt, wird.

## Claims

1. X-ray line detector with a predefined number of carrier modules (1) which have the same width (a), with a manufacturing tolerance, and are arranged in a housing (8),
wherein a one-piece printed circuit board (2), on which a photodiode (3) is arranged, is attached to each carrier module (1),
wherein this printed circuit board (2) is wider than the active area of the pixels forming the photodiode (3), wherein in each case a scintillator element (4) is attached to each photodiode (3), which scintillator element has a length (b) that exactly covers the width of the active area plus an interspace between two adjacent pixels of a photodiode (3),
wherein the width (a) of each carrier module (1) is at most twice as great as the length (b) of a scintillator element (4),
wherein the carrier modules (1) are arranged in two rows (9, 10) in the housing (8) such that the photodiodes (3) of each row (9, 10) are opposite each other,
wherein the scintillator elements (4) abut against each other upon contact at their respective end faces (28), wherein scintillator elements (4) in contact with each other are arranged in each case in opposite rows (9, 10) such that in each case one carrier module (1) of one row (9; 10), through the two carrier modules (1) of the other row (10; 9) arranged offset with respect to it, leads to scintillator element (4) being arranged against scintillator element (4).

2. X-ray line detector according to claim 1, **characterized in that** the housing (8) has a first carrier element (11) and a second carrier element (12) arranged and formed in mirror symmetry thereto, between which an inlet slot (15) for X-ray radiation is formed, wherein the carrier modules (1) are arranged alternately on these two carrier elements (11, 12) and the scintillator elements (4) are arranged in the extension of the inlet slot (15).

3. X-ray line detector according to claim 2, **characterized in that** the carrier elements (11, 12) are formed L-shaped, and in each case a groove (13) aligned parallel to the inlet slot (15) is formed in each carrier element (11, 12), in which groove (13) a lug (27) formed on the carrier modules (1) engages in a form-fit manner.

4. X-ray line detector according to one of the preceding claims, **characterized in that** each carrier module (1) is connected to the respective carrier element (11, 12) via at least one fixing element, in particular a fixing screw (14) .

5. X-ray line detector according to one of claims 2 to 4, **characterized in that** an end plate (16, 17) is arranged at each end of the housing (8), wherein the first end plate (16) is formed as a stop and the second end plate (17) is formed as a clamping element.

6. X-ray line detector according to claim 5, **characterized in that** the second end plate (17) has three clamping screws (18, 19, 20), of which the first clamping screw (18) acts in combination with the first row (9) of carrier modules (1), the second clamping screw (19) acts in combination with the second row (10) of carrier modules (1), and the third clamping screw (20) acts in combination with the scintillator elements (4).

7. X-ray line detector according to one of claims 2 to 6, **characterized in that** the housing (8) has a cover plate (25) on its side opposite the inlet slot (15).

8. X-ray line detector according to claim 7, **characterized in that** the cover plate (25) is composed of a material that, for the X-ray radiation energy used, produces scarcely any scattered radiation, in particular it is composed of carbon.

9. X-ray line detector according to one of claims 5 to 8, **characterized in that** a first buffer piece (23) is inserted between the end plate (16) and one of the two rows (9, 10) of carrier modules (1), which buffer piece (23) is half as wide as the carrier modules (1), and a first spacer (21) is inserted between the first end plate (16) and the scintillator elements (4), which spacer (21) is half as wide as the scintillator elements (4).

10. X-ray line detector according to claim 9, **characterized in that** a second buffer piece (24) is inserted between the end plate (17) and one of the two rows (9, 10) of carrier modules (1), which buffer piece (24) is half as wide as the carrier modules (1), and a second spacer (22) is inserted between the second end plate (17) and the scintillator elements (4), which spacer (22) is half as wide as the scintillator elements (4).

11. X-ray line detector according to one of the preceding claims, **characterized in that** the printed circuit boards (2) are bent and electronic components (6) are arranged in the region which is formed on the other side of the bend region (7) in relation to the photodiode (3).

12. Method for the production of an X-ray line detector with the features of one of the preceding claims with the following steps:
- inserting a first spacer (21) on a first end plate (16) and subsequently inserting a first carrier module (1') on a first carrier element (11) until the scintillator element (4) of the first carrier module (1') contacts the first spacer (21), and inserting a first buffer piece (23) on a second carrier element (12) until it contacts the first end plate (16), wherein the insertion of the first carrier module (1') and of the first buffer piece (23) can also be effected in a reversed sequence;
- alternatively to the above-named step, the first buffer piece (23) can also first of all be brought into contact with the first end plate (16) on the second carrier element (12), and then the first spacer (21) can be brought into contact with the first end plate (16), and subsequently the first carrier module (1') is brought, with its scintillator element (4), into contact with the first spacer (21);
- after one of the two alternative steps named above, a second carrier module (1") is inserted such that the scintillator element (4) thereof contacts the scintillator element (4) of the first carrier module (1') and itself contacts the first buffer piece (23) ;
- subsequently a carrier module (1) is inserted, always alternately, on the first and on the second carrier element (11, 12), wherein the respective scintillator element (4) thereof contacts the scintillator element (4) of the carrier module (1) inserted directly beforehand;
- in any sequence, a second spacer (22), which is half as wide as a scintillator element (4), is brought into contact with the scintillator element (4) of the final carrier module (1'''), and a second buffer piece (24), which is half as wide as a carrier module (1), is brought into contact with the penultimate inserted carrier module (1);
- clamping means, in particular a third clamping screw (20), are actuated in a second end plate (17) so as to press all scintillator elements (4) with the respectively opposite end faces (28) thereof against each other;
- fixing the two rows (9, 10) of carrier modules (1) with clamping means in the second end plate (17), in particular by means of a first and a second clamping screw (18, 19).

13. Method according to claim 12, **characterized in that** the individual carrier modules (1) are then fixed to the housing (8) by means of fixing means, in particular with fixing screws (14), and then the third clamping screw (20) is loosened such that there is no longer pressure acting on the scintillator elements (4).

14. Method according to claim 12 or 13, **characterized in that** a cover plate (25) is subsequently connected, in particular screwed, to the housing (8).

## Revendications

1. Détecteur de lignes à rayons X avec un nombre prédéfini de modules de support (1) présentant, jusque dans une certaine tolérance de fabrication, la même largeur (a) et disposés dans un carter (8) ;
une platine conductrice (2) d'un seul tenant sur laquelle une photodiode (3) est disposée étant placée sur chaque module de support (1) ;
cette platine conductrice (2) étant plus large que la surface active du pixel formant la photodiode (3), un élément de scintillation (4) étant respectivement placé sur chaque photodiode (3), cet élément recouvrant dans sa longueur (b) exactement la surface active dans sa largeur par rapport à un espace intermédiaire entre deux pixels connexes d'une photodiode (3) ;
la largeur (a) de chaque module de support (1) étant de tout au plus le double de la longueur (b) d'un élément de scintillation (4) ;
les modules de support (1) étant disposés dans deux rangées (9, 10) dans le carter (8) de telle sorte que les photodiodes (3) de chaque rangée (9, 10) se fassent face ;
les éléments de scintillation (4) butant les uns contre les autres sur la butée au niveau de leurs côtés avant (28) respectifs ;
les éléments de scintillation (4) butant les uns contre les autres étant disposés dans des rangées (9, 10) respectivement opposées, de sorte que respectivement un module de support (1) d'une rangée (9 ; 10) permette, à travers les deux modules de support (1) disposés de façon décalée par rapport à lui de l'autre rangée (10 ; 9), que l'élément de scintillation (4) soit disposé contre l'élément de scintillation (4).

2. Détecteur de lignes à rayons X selon la revendication 1, **caractérisé en ce que** le carter (8) comporte un premier élément de support (11) et un deuxième élément de support (12) disposé et réalisé de façon symétrique à l'axe par rapport à lui entre lesquels une fente d'entrée (15) pour rayonnement X est réalisée, les modules de support (1) étant disposés de façon alternée sur ces deux éléments de support (11, 12) et les éléments de scintillation (4) étant disposés dans le prolongement par rapport à la fente d'entrée (15).

3. Détecteur de lignes à rayons X selon la revendication 2, **caractérisé en ce que** les éléments de support (11, 12) sont en forme de L et qu'une rainure (13) orientée parallèlement à la fente d'entrée (15) est respectivement réalisée dans chaque élément de support (11, 12) dans laquelle un bec (27) réalisé au niveau des modules de support (1) s'engrène par complémentarité de formes.

4. Détecteur de lignes à rayons X selon l'une quelconque des revendications précédentes, **caractérisé en ce que** chaque module de support (1) est relié, via au moins un élément de fixation, notamment une vis de fixation (14), à l'élément de support (11, 12) respectif.

5. Détecteur de lignes à rayons X selon l'une quelconque des revendications 2 à 4, **caractérisé en ce que** respectivement une plaque d'extrémité (16, 17) est disposée au niveau des extrémités du carter (8), la première plaque d'extrémité (16) étant réalisée sous la forme d'une butée et la deuxième plaque d'extrémité (17) étant réalisée sous la forme d'un élément de serrage.

6. Détecteur de lignes à rayons X selon la revendication 5, **caractérisé en ce que** la deuxième plaque d'extrémité (17) comporte trois vis de serrage (18, 19, 20) parmi lesquelles la première vis de serrage (18) interagit avec la première rangée (9) de modules de support (1), la deuxième vis de serrage (19) interagit avec la deuxième rangée (10) de modules de support (1) et la troisième vis de serrage (20) interagit avec les éléments de scintillation (4).

7. Détecteur de lignes à rayons X selon l'une quelconque des revendications 2 à 6, **caractérisé en ce que** le carter (8) comporte une plaque de recouvrement (25) au niveau de son côté opposé à la fente d'entrée (15).

8. Détecteur de lignes à rayons X selon la revendication 7, **caractérisé en ce que** la plaque de recouvrement (25) est réalisée à partir d'une matière ne produisant, pour l'énergie de rayonnement X utilisée, presque aucun rayonnement dispersé, notamment fabriquée en carbone.

9. Détecteur de lignes à rayons X selon l'une quelconque des revendications 5 à 8, **caractérisé en ce qu'**une première pièce tampon (23) est insérée entre la plaque d'extrémité (16) et une des deux rangées (9, 10) de modules de support (1), cette pièce étant moitié moins large que les modules de support (1) et une première pièce d'écartement (21) étant insérée entre la première plaque d'extrémité (16) et les éléments de scintillation (4), cette pièce étant moitié moins large que les éléments de scintillation (4).

10. Détecteur de lignes à rayons X selon la revendication 9, **caractérisé en ce qu'**une deuxième pièce tampon (24) est insérée entre la plaque d'extrémité (17) et une des deux rangées (9, 10) de modules de support (1), cette pièce étant moitié moins large que les modules de support (1) et qu'une deuxième pièce d'écartement (22) est insérée entre la deuxième plaque d'extrémité (17) et les éléments de scintillation (4), cette pièce étant moitié moins large que les éléments de scintillation (4).

11. Détecteur de lignes à rayons X selon l'une quelconque des revendications précédentes, **caractérisé en ce que** les platines conductrices (2) sont coudées et que des composants électroniques (6) sont disposés dans la région réalisée sur l'autre côté de la zone d'inflexion (7) par rapport à la photodiode (3).

12. Procédé de fabrication d'un détecteur de lignes à rayons X avec les caractéristiques d'une des revendications précédentes avec les étapes suivantes :
- insertion d'une première pièce d'écartement (21) au niveau d'une première plaque d'extrémité (16) puis insertion d'un premier module de support (1') au niveau d'un premier élément de support (11) jusqu'à ce que l'élément de scintillation (4) du premier module de support (1') bute contre la première pièce d'écartement (21) et insertion d'une première pièce tampon (23) au niveau d'un deuxième élément de support (12) jusqu'à buter contre la première plaque d'extrémité (16), l'insertion du premier module de support (1') et de la première pièce tampon (23) pouvant être effectuée dans l'ordre inverse ;
- en variante par rapport à l'étape susmentionnée, la première pièce tampon (23) au niveau du deuxième élément de support (12) peut d'abord être amenée en butée contre la première plaque d'extrémité (16) puis la première pièce d'écartement (21) peut être amenée en butée contre la première plaque d'extrémité (16), à la suite de quoi le premier module de support (1') est amené en butée avec son élément de scintillation (4) contre la première pièce d'écartement (21) ;
- après une des deux étapes alternatives susmentionnées, un deuxième module de support (1") est inséré de telle sorte que son élément de scintillation (4) bute contre l'élément de scintillation (4) du premier module de support (1') et que lui-même bute contre la première pièce tampon (23) ;
- un module de support (1) est ensuite toujours inséré alternativement au niveau du premier et du deuxième élément de support (11, 12), leur élément de scintillation (4) respectif butant contre l'élément de scintillation (4) de leur module de support (1) utilisé directement avant ;
- suivant une séquence quelconque, une deuxième pièce d'écartement (22) moitié moins large qu'un élément de scintillation (4) est placée en butée contre l'élément de scintillation (4) du dernier module de support (1''') et une deuxième pièce tampon (24) moitié moins large qu'un module de support (1) est placée en butée contre l'avant-dernier module de support (1) utilisé ;
- des moyens de serrage sont actionnés dans une deuxième plaque d'extrémité (17), notamment une troisième vis de serrage (20), de telle sorte que tous les éléments de scintillation (4) soient comprimés les uns contre les autres avec leurs surfaces frontales (28) respectivement opposées ;
- fixation des deux rangées (9, 10) de modules de support (1) à l'aide de moyens de serrage dans la deuxième plaque d'extrémité (17), notamment au moyen d'une première et d'une deuxième vis de serrage (18, 19).

13. Procédé selon la revendication 12, **caractérisé en ce que** dans la borne, les modules de support (1) individuels sont fixés au carter (8) à l'aide des moyens de fixation, notamment à l'aide de vis de fixation (14), et qu'ensuite, la troisième vis de serrage (20) est verrouillée, de sorte qu'aucune pression n'agisse plus sur les éléments de scintillation (4).

14. Procédé selon la revendication 12 ou 13, **caractérisé en ce qu'**ensuite, une plaque de recouvrement (25) est reliée, notamment vissée, au carter (8).
